# EUROPEAN PATENT SPECIFICATION

(11) **EP 2 893 321 B1**
(45) Date of publication and mention of the grant of the patent: **24.02.2021**
(21) Application number: 12773111.5
(22) Date of filing: 03.09.2012
(51) Int. Cl.: G01N 21/84, G01N 33/00, G01N 33/22

(54) **COMPACT SIZE EXPLOSIVES DETECTOR AND METHOD FOR DETECTING EXPLOSIVES**
KOMPAKTER EXPLOSIVSTOFFDETEKTOR UND VERFAHREN ZUR ERMITTLUNG VON EXPLOSIVSTOFFEN
DÉTECTEUR D'EXPLOSIFS À TAILLE COMPACTE ET PROCÉDÉ DE DÉTECTION D'EXPLOSIFS

(43) Date of publication of application: 15.07.2015
(73) Proprietor: RS Dynamics GmbH, 6304 Zug (CH)
(72) Inventor: BLAHA, Jiri, 6315 Oberägeri (CH); DUBAC, Jan, 148 00 Praha 4 (CZ); ZÁSTERA, Michal, 182 00 Praha 8 (CZ); MÁZL, Roman, 160 00 Praha 6 (CZ)
(74) Representative: Schirbach, Marcel
(86) International application number: PCT/IB2012/001693
(87) International publication number: WO 2014/033493

(56) References cited:
- WO-A2-2007/002037
- US-A1- 2003 085 348
- US-A1- 2011 203 931

## Description

### Field of the Invention

This invention relates to a pocket size explosive detector according to the preamble of independent claim 1.

Furthermore, this invention also relates to a method for detecting explosives according to the preamble of independent claim 13.

### Background of the Invention

Current technologies used for detection of explosive traces are obviously based on direct air sampling of explosive vapors surrounding the found explosive contraband or particulate sampling technique. The direct air sampling is effective only for sufficiently volatile explosive components, while the explosive components, mostly used for creation of so called plastic explosives feature very low or zero volatility at ambient temperature.

There is a great demand world-wide for reliable contact-less detection technologies, being capable to sample and detect all kinds of explosive compounds, mainly widely used non-volatile or home-made explosive agents.

Most of the current detection technologies use an IMS (Ion Mobility Spectrometry) detection principle, or other technologies, focused for direct identification of the sample; unfortunately, those detection principles are very sensitive for cross-talks by various disturbing chemicals and saturation due to very limited dynamic range. Both above limitations create potentially high false alarm rate and causes moreover a number of serious problems with after-exposure cleaning of the system.

The current portable systems work mostly in the cycle "sampling - pre- concentration - analyses". This operation cycle requires the operator to sample just from one spot, in the good believe this spot contains the found traces. There is no indication about the properly selected spot during the sampling interval, so it frequently happens to sample from the improper spot or location, while losing a valid operational time.

Most of the current systems, utilizing various detection technologies, namely IMS, are quite complicated, climatic conditions-sensitive instruments, not very suitable for heavy-duty field and/or military application. Also, they require advanced level of operator's qualification and demanding training of the operator.

Most of today's operators require to scan the controlled subjects (parcels, baggage, people) only in vapor mode, that is under standard sampling condition practically not successful namely in the case of plastic explosives (and all other nonvolatile explosives), because those substances create no vapors at normal ambient temperature and that is why no vapor traces can be sampled/ detected in standard vapor mode.

Systems and methods for reliable detection of various explosives are urgently needed and are now at the forefront of many research affords. The best wanted detection system should be extremely reliable, sensitive, the smallest sized, operating preferably only in vapor mode, resistant against cross-talking chemicals to avoid false alarms and very simple for operation not to require extensive training and special education of the operator. Under the standard environmental conditions, the detection of explosives is very complicated, complex task, especially if we consider detection of all branch of non-volatile types of explosives, disturbing factors of the variety of environmental condition (humidity, dust, temperature). Moreover, interferences from various chemicals, human sweat, various solvents, e.t.c. lead to false alarms, that are obviously difficult to distinguish from actual positive detection.

The wide variety of techniques, used to sample explosives include manual swiping of the scanned subject in so called particulate mode or sampling the air, surrounding the scanned subject at ambient temperature and consequent preconcentration in a good believe to collect the most of the traces from the vicinity of the scanned subject. The manual swiping is not very popular or sometimes difficult to apply, air sampling requires huge amount of air to be sampled and pre-concentrated to collect enough volume of traces to satisfy the system detection limit.

The variety of techniques, used to detect/analyze explosives, based on ion mobility spectrometry, IR spectroscopy, micro-wave spectroscopy, Raman or fluorescence spectrometry bring good detection limits, however all those principles feature low dynamic range and are very sensitive to cross-talks, caused by interfering chemicals, overloads and suffer from difficult cleaning if exposed by huge sampled concentration. Portable detection system built on above principles are usually complicated, big, heavy and not operationally robust enough to work reliably in the demanding field conditions, characterized by changing the humidity, temperature, dust and rough handling by the operator. High level of operator's training and adequate operator's education is obviously necessary for successful detection using above technologies, that limits the range of people usable to make the detection job. US 2004/053421 A1 discloses inspection systems that scan luggage and cargo to detect residues of explosives. The system comprises inter alia an infrared laser adapted to emit light, an optical system adapted to deliver a beam of the laser light to illuminate an interrogation area of the surface, the illumination having sufficient intensity and duration to cause selective desorption of molecules of the explosive substance present on the surface without substantially damaging the surface, and at least a portion of the molecules being thermally decomposed to produce NO2 molecules, a collection system having an aperture and being adapted to collect at least a portion of the desorbed molecules through the aperture, a reaction cell in communication with the collection system, the reaction cell having a reaction zone, an inlet for receiving in the reaction zone the molecules collected by the collection system, an outlet for release of the air, and the reaction cell containing an alkaline, aqueous luminol-containing solution, a light detector to detect light produced chemiluminescently by a chemical reaction between the luminol and the NO2 within the reaction cell and to output an electrical signal indicative of the detection of the light, and signal means for indicating the presence of the NO2 produced by the decomposition of the contraband substance, the signal means being operably connected to the light detector and responsive to the receipt of the electrical signal. In one aspect, the means for decomposing the desorbed molecules comprise a pyrolyzer which may be a heated Pt-Rh alloy wire.

### Summary of the Invention

It is an object of the present invention to provide a pocket size explosive detector and appropriate method therefor, not having the disadvantages of the prior art, in particular, a pocket size explosive detector with a simpler construction and easier handling compared to the state of the art. The problem underlying the invention for the pocket size explosive detector is solved by the features of independent claim 1; the problem underlying the invention for the mothod for detecting explosives using the pocket size detector of claim 1 is solved by the features of independent claim 13. The current invention relates to a system and methodology for ultra-fast, highly sensitive, continuous vapor-mode detection of explosives.

The present disclosure further relates to the technical design and sub-miniature construction of the system, using Infra-Red (further IR) Continuous Sampling technique, together with newly invented thermal decomposition detection unit, integrated-in one body with Infra-Red sampler.

In the new embodiment, the newly designed detection system, according to this invention, is built in very compact, pocket sized and light-weight body, especially shaped for the whole-day wearing on the policeman belt.

In another embodiment, the new system works in direct Infra-Red continuous Vapor Mode with instant reading, not requiring sampling, preconcentration and analyzing period.

The invention uses new flat IR source transmitting the IR radiation directly to the scanned surface for ideal release of all volatile and also nonvolatile explosive traces.

In yet another embodiment, the IR sampler is integrated into one body with thermal decomposition unit for fast detection, minimizing the losses of the scanned traces volume and minimizing the battery energy consumption.

In yet another embodiment, the thermal decomposition unit, directly integrated into one body with IR radiation source, is composed by a silica-glass tube with on-surface integrated non-linear heating element to achieve minimal temperature gradient along the length of the thermal decomposition tube and precision regulation of the operating temperature.

In yet another embodiment, the thermal decomposition unit is housed into a special thermal insulating cylinder for optimal shape of the temperature gradient and minimizing the temperature losses.

In yet another embodiment, the new detection cartridge with a sunk molecular membrane is used for reliable and long-term stable detection of photons as a response to the decomposed explosive molecules coming into reaction with detection liquid.

In yet another embodiment, the shape of the detection cartridge is especially designed to achieve the widest possible range of 3-D working angels of the cartridge, to achieve the detection membrane to be sank under the detection liquid level within the widest operational angles.

In yet another embodiment, the gas output of the thermal decomposition unit is connected with the detection cartridge using an especially designed needle line-up, enabling fast and reliable connection and/or change of the detector cartridge.

In yet another embodiment, the new detection cartridge consists of the main cartridge body, housing the special detection liquid, still sank detection polymer membrane, sensing cap, creating detection annular chamber for convolute circulation of the detected gas along the dry membrane surface together with proper connection to the connecting needles, and the glossy optical window for detecting photons, created at the wet side of the membrane to pass through the layer of the detection liquid and through this window to the optical single photon detection unit.

In yet another embodiment, the construction shape of the detection cartridge is designed for the tightest possible displacement and optical connection to the single-photon optical detection unit.

In yet another embodiment, the construction shape of the detection cartridge is designed for the one-movement easy cartridge deployment to the connection needles line-up and easy replacement of the used cartridge.

In yet another embodiment, the detection cartridge is equipped with a special pulling handle for easy manipulation and removing that from it's slot.

In yet another embodiment, the detection cartridge is equipped with soft plastic optical shield to avoid penetration even of the weakest interfering light into the cartridge.

In yet another embodiment, the detection cartridge is inserted into a rectangular slot, created inside the one-body instrument frame, housing also a newly developed single photon optical detection unit.

In yet another embodiment, the one-body instrument frame contains two slots - one for the detection cartridge and the other one for the single-photon optical detection unit to secure minimal distance between photo sensing element of the single-photon optical detection unit and the detection cartridge optical window.

In yet another embodiment, the one-body instrument frame creates the holding slot with silent-block vibration insulation lodgement for the vibration pump installation

In yet another embodiment, the one-body instrument frame creates the mounting points for the 3-D montage of the 3-D Flex PC board, containing all the system electronics, communication connectors and battery connectors, all at one Flex PC board.

In yet another embodiment, the one-body instrument frame contains the mounting points for connecting the front analytical part containing the Infra-Red continuous sampler and thermal decomposition detection unit.

In yet another embodiment, the one-body instrument frame contains one system mounting point for fixing the whole instrument formation into the rectangular-tube-shaped explosive detector case, using one screw only.

In another embodiment, the explosive detector case is made of high-density lightweight carbon-fiber plastic to achieve extreme durability and robustness.

In still another embodiment, the newly designed single-photon detection unit, according to this invention, is used to achieve the highest level of system sensitivity.

In still another embodiment, the new system is equipped with built-in Wi-Fi module, placed on common 3-D Flex PC board, for wire-less remote control and data transfer.

In still yet another embodiment, the powerful multi-processor built-in computer and operational software was designed to control all the operations and to achieve easy user friendly operation.

In still yet another embodiment, only one button is used to operate all the system functions due to intelligent, user-friendly operational software.

In still yet another embodiment, the full graphic, color screen is used to provide transparent, user friendly communication and detection results to the operator

In still yet another embodiment, the newly developed software is used for easy wire-less remote control for robotic application.

In still yet another embodiment, the newly developed software is used for remote Internet factory checking and first-level factory repair of the each system.

In still yet another embodiment, the special belt holster is designed for convenient and dexterous wearing/using the unit in the field.

In yet another embodiment, four special LiPol battery cells are used as the main system rechargeable battery, housed in robust carbon-fibers battery case following the shape of the whole unit.

The main advantage of this invention is the system and methodology for detection of explosives, more particularly using Infra-Red semi-selective sampling technique built into ultra-miniature pocket-sized, ultra-fast detection system. The analytical and detection device according to the invention is equipped with various ways to communicate with surroundings. For manufacturer set up, calibration and software update, the standard RS232 port (located at module 44) is provided, using the advantages of simplicity and reliability of the communication protocol. The customer can use either standard USB 2.0 port 44, with standard micro-USB connector and cable to connect the unit to PC, or built-in WiFi module 45 for convenient remote data transfer and/or full remote control operation and Internet remote communication.

Special remote control software for PC and also for mobile ANDROID platform, using the wire-less Wifi connection, have been developed for convenient application of the detection and analytical system according to the invention, for outdoor/indoor field robotic application according to the invention.

The test results prove, that the cost effective, sensitive enough and simple, very portable and fast detection and analytical system has been created for quantitative detection of explosive traces in the presence of other organic material by combining the intelligent Infra-Red sampling with fast and sensitive thermal decomposition and optical detection/analytical unit. The developed system according to the current invention, is technically simple and provides clear data of presence and concentration of the explosive materials not disturbed by presence of the strong concentration of cross-talking organic chemical components. Even if detailed chemical identification of the explosive root components is not provided, this simple, fast and pocked sized system finds number of customers who calls for easy to operate, very sensitive, light-weight and operationally robust detection/analytical system.

### Brief Description of the Drawings

These and other features and advantages of the present invention will be better understood by reference to the following detailed description when considered in conjunction with the accompanying drawing wherein:

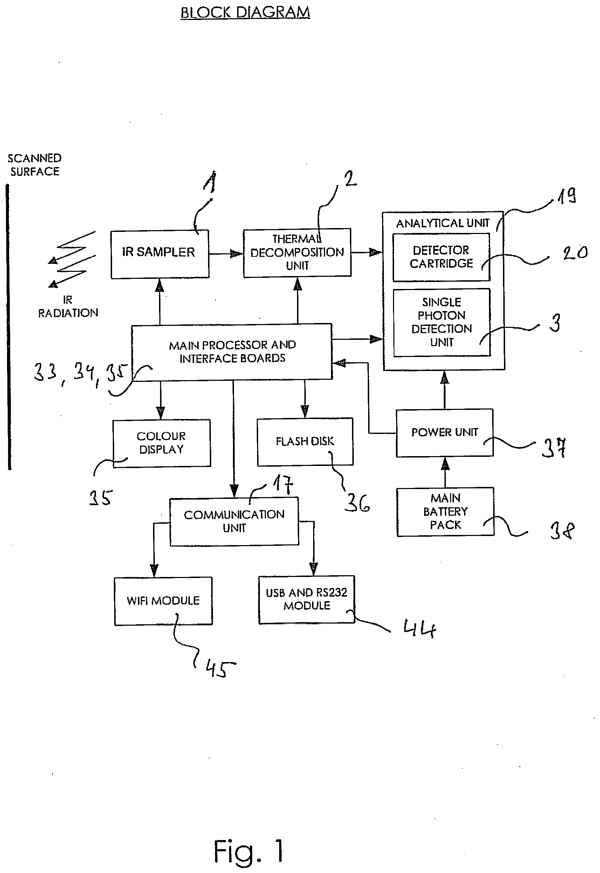

Fig. 1 provides a block diagram of an exemplary explosive detector and particularly the detection system with Infra-Red sampler, thermal decomposition unit, analytical unit, main processor and interface units with consequent control and data acquisition parts and power control and supply unit.

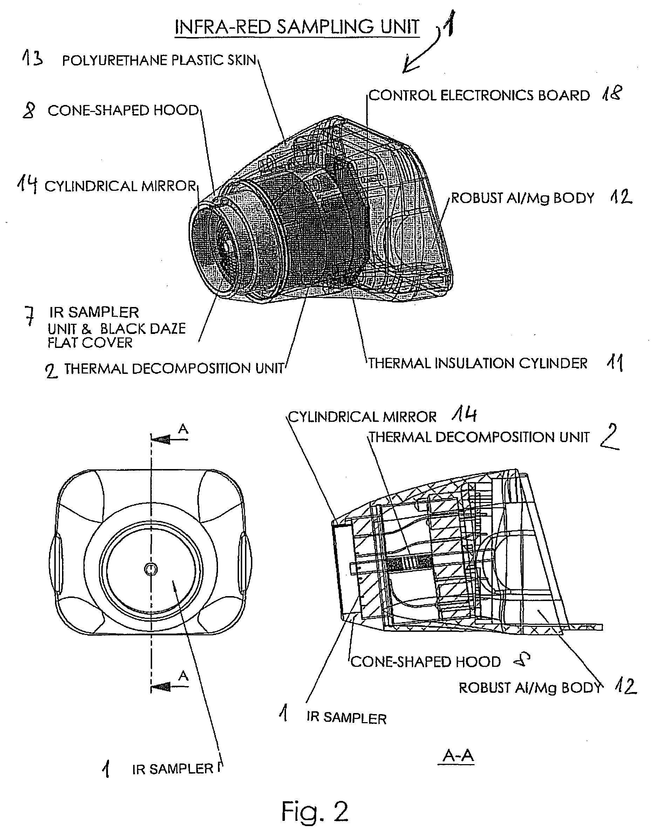

Fig. 2 provides the drawing of the composition of the Infra-Red sampler and thermal decomposition unit, together with control electronics creating thus one building block of the exemplary explosive detector according to the invention, where in the upper part of Fig 2 is the axonometrical view of the Infra-Red sampler with the termal decomposition unit, together with control electronics, all in one formation. In the lower part of Fig. 2, there is the front view of the Infra-red sampler on the left side and cross-section A-A of the whole formation on the lower right side.

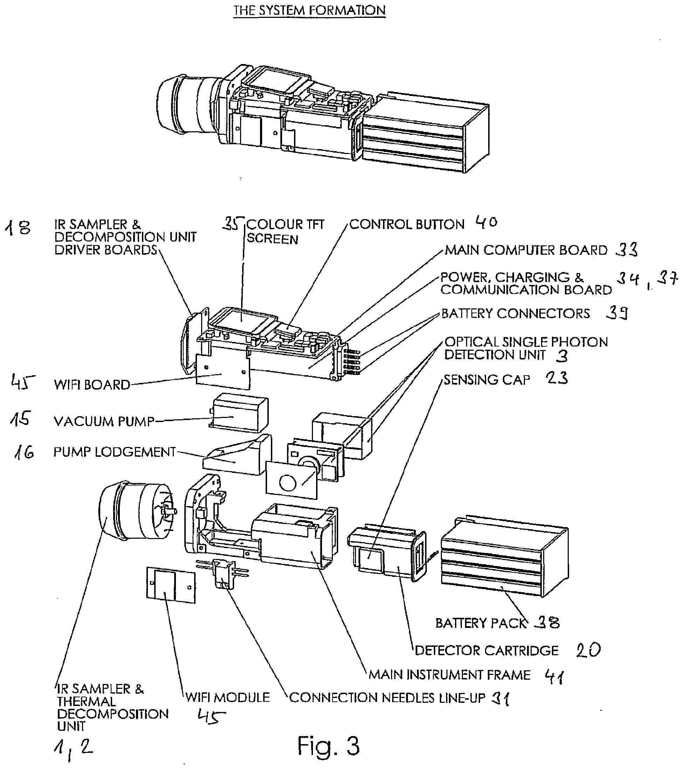

Fig. 3 provides a drawing of the formation of all the other operational units of the exemplary explosive detector according to the invention, where on the upper part of the Fig.3 there is the drawing of the whole formation and in the lower part of the Fig. 3, there is a dissassembled view of the whole formation, showing all the individual parts of the formation and their positioning.

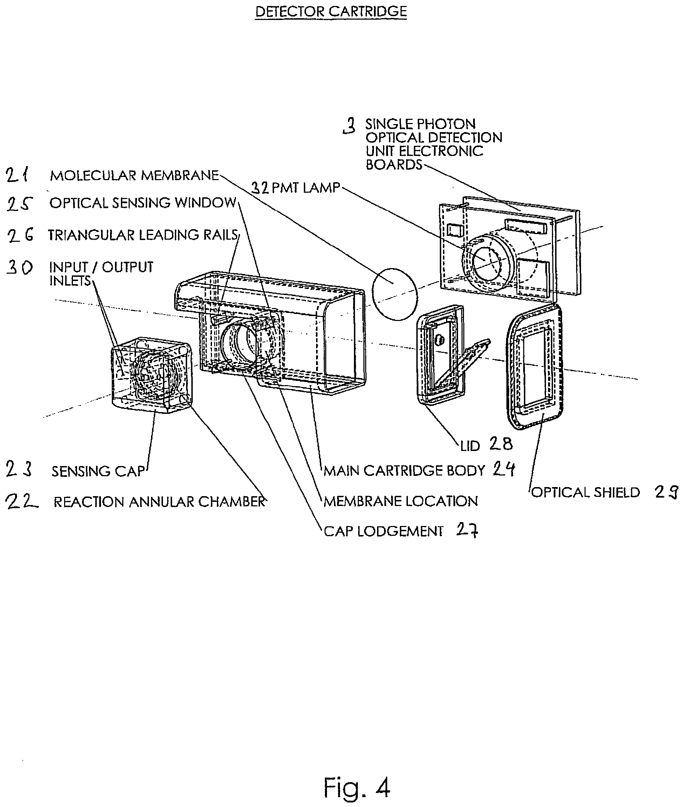

Fig. 4 provides the drawing of the detector cartridge together with the single photon optical detection unit, in dissasembled view.

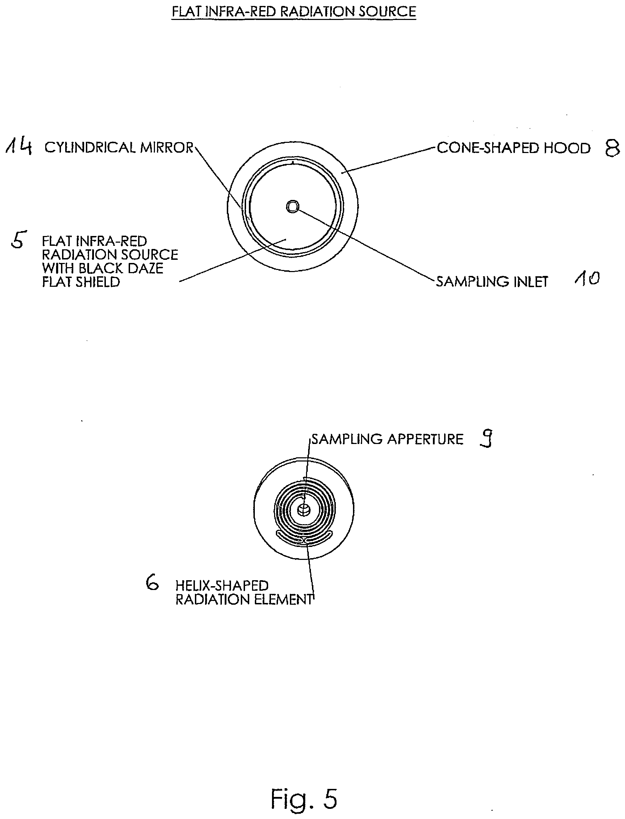

Fig. 5 provides a drawing of the flat Infra-Red radiation source helix-shaped element having an areal shape for securing the areal homogeneuous radiation of the Infra-Red energy. In the upper part of Fig. 5 there is the front view of the Infra-Red radiation source and at the lower part of Fig.5, there is the axonometric view of the helix-shaped radiation element.

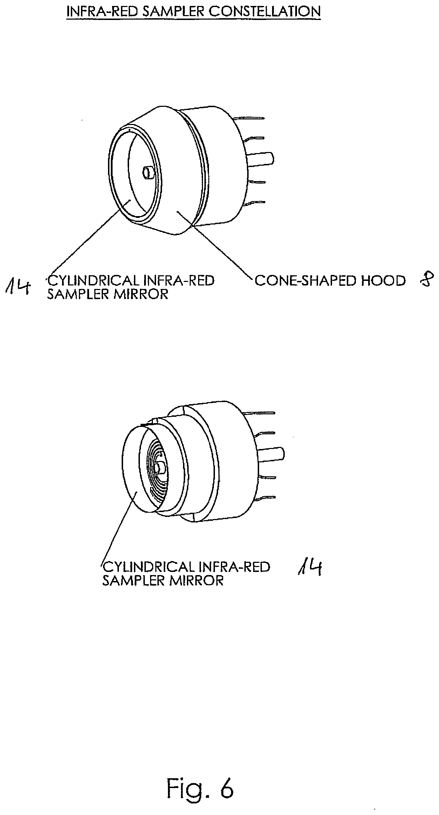

Fig. 6 provides the drawing of the cylindrical Infra-Red sampler mirror, providing the increase of the efficiency of the Infra-Red radiation, where in the upper part of Fig. 6. there is the view of the whole constellation of the Infra-red smapler and, in the lower part of Fig. 6 there is a view of assembled individual parts with the cone-shaped hood removed.

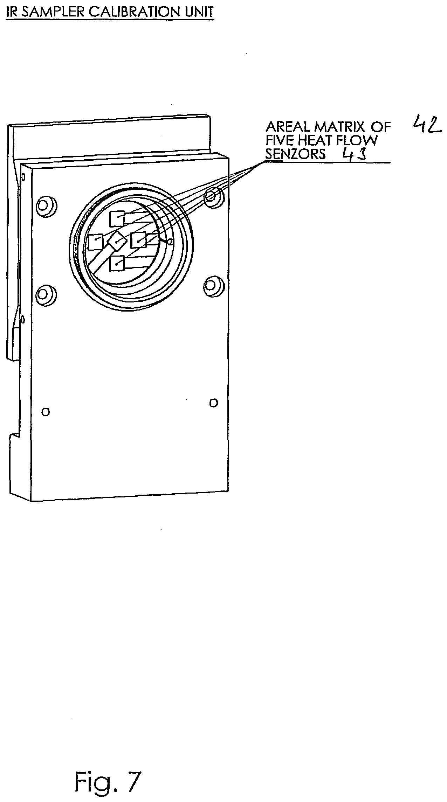

Fig. 7 provides the drawing of IR sampler calibration unit with five heat flow detectors.

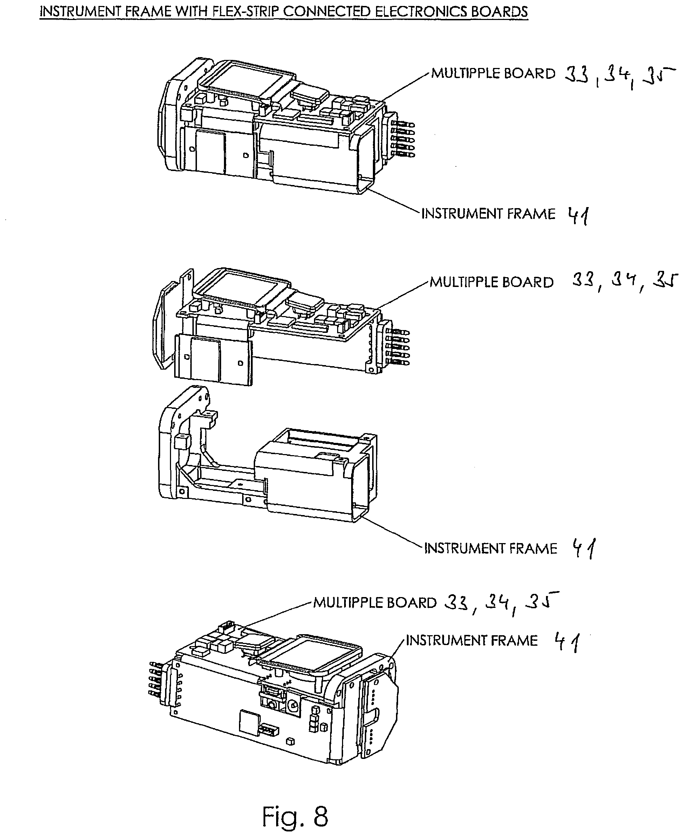

Fig. 8 provides a drawing of the instrument frame with multipple processor board, where on the upper part of Fig. 8, there is an axonometric view of the the whole formation of instrument frame with multipple procesor board, the middle part of Fig. 8 shows the disassembled view of the mentioned fomation and the lower part of Fig. 8 shows the right side view of the assembled formation.

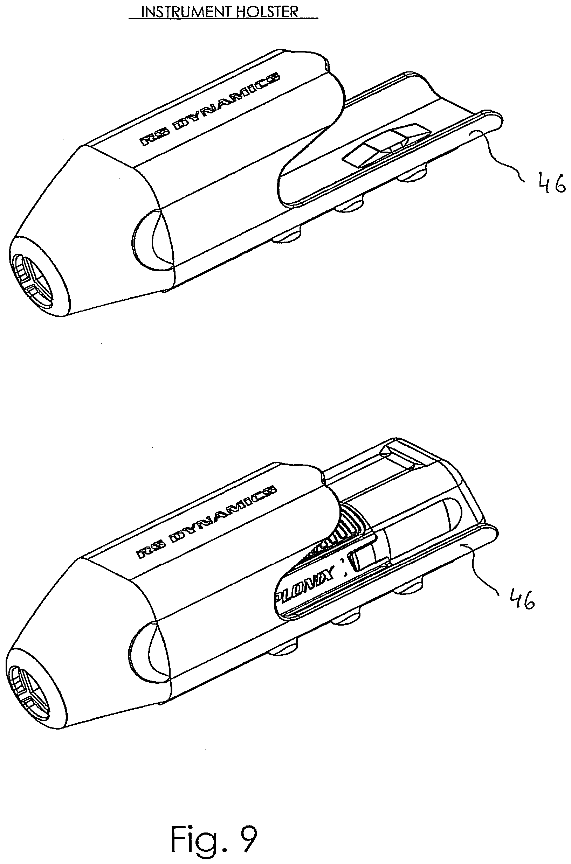

Fig. 9 shows the explosive detector carrying and protecting holster, where in the upper part of Fig. 9, there is a view of the empty holster and in the lower part of Fig. 9, there is a view of the holster, carrying the explosive detector.

### Detailed Description of the Invention

Unless defined otherwise, all the technical and scientific terms used herein have the same meaning as commonly understood to one of ordinary skills in the art to which this invention belongs.

Although conventional techniques have taken a multidimensional approach to explosive detection and analyses, obviously using gas chromatography followed by IMS detection unit or using the IMS system as the only detection part, these prior art techniques have been focused on addressing the unique problems in fast, continuous sampling and detection of namely non-volatile or very low vapor pressure explosives, using continuous vapor mode detection technique with Infra-Red sampling of explosive traces.

The current invention addresses the technical solution and operating methodology of the Infra-Red continuous mode sampler 1 together with integrated-in thermal decomposition unit 2 and consequent single photon optical detection unit 3 and intelligent data acquisition and control unit 4, communication unit 17 to create the ultra-fast, highly sensitive and false alarm extremely resistant explosive detection system, capable of instant sampling and detection of even non-volatile explosives in real-time instant vapor mode. The whole detection system, including all the sampling, detection and analytical components is designed with the aim to achieve the sub-miniature construction and low-cost high volume serial production to create affordable, simple to operate explosive detector for everyday use of the operator with no need of special qualification and/or training.

### Sampling system

Turning to the sampling system, the new system according to the invention uses Infra-Red radiation energy of the selected wavelength to semi-selectively address explosive materials molecules, for their preferentially release from solid to vapor phase (Fig.1).

According to the invention the sampling system consists of especially designed flat Infra-Red radiation source 5 (Fig. 2, 5), emitting the generated Infra-Red energy homogeneously from it's flat surface to the scanned surface.

In a particular embodiment, the flat Infra-Red radiation source consists of the helix-shaped radiation element 6 (Fig. 5), with modified helix-shape, according to the invention, described on the Fig. 5, to achieve the maximal areal homogeneity of the radiated Infra-Red energy.

In the yet another embodiment, the flat Infra-Red radiation source 5 is covered with two-layers thin-film black daze shield (cover) 7 (Fig. 2) to allow mostly Infra-Red radiation to pass through the shield and, also, to protect the radiation source 5 against mechanical damage.

In yet another embodiment, the black daze flat Infra-Red source shield 7 is equipped with cone-shaped hood 8 (Fig. 5) for shielding the sampling process against the ambient blowing wind and the central circular aperture 9_(Fig. 5) for the sampling silica-glass inlet 10 to pass through.

In still yet another embodiment, the set of flat Infra-Red source 5, together with it's shield 7 according to the point [0066], is mechanically fixed at the temperature insulation cylinder with the central cylindrical room for the emplacement of the thermal decomposition unit 2. (Fig. 2).

In yet another embodiment, the set of Infra-Red radiation source 5, according to the points [0063 - 0066], is housed into the robust, molded aluminum/ magnesium body 12_(Fig. 2) for achieving operational robustness and good temperature dissipation.

In yet another embodiment, the molded aluminum/ magnesium body 12 is covered with polyurethane plastic skin 13 (Fig. 2) to avoid burning up the operator's fingers and to dump possible mechanical impacts when the explosive detector would be dropped down to the hard surface.

In yet another embodiment, the front part of the IR sampler 1, created according to the points [0062 - 0067] is equipped with the cone-shaped hood 8 (Fig. 2, 5), creating thus the pneumatically closed area for successful sampling of the explosive traces from the scanned surface.

In another embodiment, the cone-shaped hood 8 (Fig. 2, 5) is made from highly chemically inert and temperature resistant material "PEEK".

In yet another embodiment, the inner cylindrical surface of the cone-shaped hood 8 (Fig. 6) is equipped with the highly polished stainless-steel cylindrical IR sampler mirror 14 to improve the homogeneity of the areal Infra-Red radiation and for increasing the effectiveness of the radiation.

In yet another embodiment, the completed formation of the Infra-Red sampler 1 together with thermal decomposition unit 2 (Fig. 2) is mounted directly to the terminal flex-part of the main PC board without any connectors to secure the highest possible reliability of the system.

### Analytical and detection system

As shown in the block diagram provided in Fig. 1 and drawings at Fig. 2, once the traces of explosives from the scanned surface are released and converted to the vapor phase using the Infra-Red radiation energy, the obtained gas sample is directly sucked by integrated vacuum pump 15 (housed in the neoprene pump lodgement 16), into the thermal decomposition unit 2 (Fig. 2), having the gas input located directly in the center of the flat Infra-Red radiation source 5 (Fig. 2, 5). In this configuration, all the parts operate at the accurate temperature to avoid any condensation of the sampled components and features no loses of the sample as the detection/analytical part is in fact housed just behind the flat Infra-Red radiation source 5 (Fig. 2) and kept at the accurate operational temperature all the operational time.

In an exemplary embodiment, the thermal decomposition unit 2 is composed by the silica-glass tube with processed inner surface and the non-linear heating element located on the outer surface in the middle of the length of the tube.

In yet another embodiment, the heating element is composed by a heating wire, wound in non-linear way along the axes of the tube, compensating thus opposite non-linear temperature drops given by location of the tube inside the temperature insulating cylinder 11 to achieve gradient-less distribution of the operating temperature along the decomposition tube.

In yet another embodiment, the heating wire is driven using PWM mode for precise regulation of the thermal decomposition unit 2 (Fig. 2) temperature.

In yet another embodiment, the heating wire is used as a temperature sensing element during the power pauses of the PWM cycle. This arrangement secures the tightest sensing element coupling to the mass of the silica-glass tube, securing thus the most precise temperature measurement and regulation of the inner tube surface temperature for the most accurate operation of the thermal decomposition unit 2 provided by the integrated control electronic board (unit) 18. The whole formation is housed into the robust AL/Mg body 12.

In yet another embodiment, the heating wire is fixed in it's non-linear displacement and operating position along the tube by a special high-temperature resistant cement.

The decomposed sample is further transferred into the analytical unit 19, consisting of detector cartridge 20 (Fig. 1, 4) with in-detection-liquid sunk molecular membrane 2'I (Fig. 4), reaction annular chamber 22 (Fig. 4) created inside the sensing cap 23 (Fig. 3, 4) and the optical single photon detection unit 3 (Fig. 4).

In exemplary embodiment the detector cartridge 20 (Fig. 4) consists of the main cartridge body 24, made from transparent and high pH resistant plastics, having the shape according to the invention, shown in details at the Fig. 4.

As shown at Fig. 4, the detector cartridge 20 is built in vertical orientation, having the place of the detection membrane 21 located at the left side (Fig. 4), with the position securing the maximal range of operating explosive detector angles, in which the membrane 21 is fully sunk under the level of detection liquid. The fully sunk membrane 21 (Fig. 4) represents the fundamental condition for the proper operation of the whole detection system and it's detection stability.

As also shown at Fig. 4, the detector cartridge 20 is equipped with the optical sensing window 25, located at the opposite site to the sunk site of the membrane 21 to minimize the length of the optical pass for transferring the generated photons to the optical sensing element of the single photon detection unit 3 (Fig. 4).

As visible at Fig. 4, the detection cartridge is equipped with four triangular leading rails 26 for inserting the sensing cap 23, cap lodgement 27 for sealed positioning of the sensing cap 23, and the covering back lid 28 together with optical shield 29.

In exemplary embodiment, the sensing cap 23 is embattled using the leading rails 26 onto the left side of the detector cartridge 20, providing thus the connection between the thermal decomposition unit 2 (Fig. 2) and the detection membrane 21 (Fig. 4) by means of the input/ output inlets 30, and, also the reaction annular chamber 22 (Fig. 4), featuring the proper volume for the adequate gas lavation around the dry membrane surface, to achieve the requested system time constant and operational dynamics.

In another embodiment, the sensing cap 23 (Fig. 4) is manufactured from the flexible plastics, securing the adequate gas and liquid sealing of the cap 23 along it's lodgment 27 (Fig. 4), created at the left side of the detector cartridge 20 (Fig. 4) and being chemically resistance against the measured media and/or the detection liquid.

In yet another embodiment, the sensing cap 23 contains two input/output inlets 30 for connecting the gas input/output connection needles line-up 31 (Fig. 3)

The single photon detection unit 3 (Fig. 4) consists of especially developed sub-miniature photomultiplier (PMT) lamp 32, sensing analogue electronics, high voltage source and control processor, that controls the operation of the whole single photon detection unit 3 and provides the first level of the intelligent digital signal processing, converting the photomultiplier analogue signal to the digital stream, with digital noise reduction and automatic baseline handling.

The digital output of the single photon detection unit 3 (Fig. 4) is further processed by the system computer located at the main processor and interface board 33, which right side part contains the power, charging and communication board 34 (Fig. 3), with the output, displayed at the color screen 35 (TFT display) (Fig. 3) either in numerical and graphic form and, also, stored at the main system flash disk 36. The main processor and interface board 33 is connected by flex part the power unit 37 and main battery pack 38 (connected by high current battery connectors 39) supplying the whole electronic system. The operating of the system is manually controlled by operator by pressing the control button 40 (Fig. 3) located at the upper part of the whole explosive detector. The whole constellation of above described electronic boards are located inside the unit by fixing along the main instrument plastic frame 41.. (Fig. 3, 8). The completed explosive detector is equipped with plastic holster 46 protecting the explosive detector and securing comfortable wearing the unit by operator.

### Calibration of the analytical system

The detection and analytical system according to the invention is calibrated using a especially developed calibration device in lieu of certified standards by exposing the detection and analytical system to the known concentration of the known explosives, such as through well characterized solid particles. The especially developed calibration device works using the principle of computer controlled continues thermal vapor phase generator, creating defined flow of defined concentration of the explosive sample in defined number of consequent steps of concentrations, covering thus the whole dynamic range of the detection and
analytical system. Multiple thermal vapor phase generators are consequently used to cover the whole wide spectrum calibration for all the existing explosive materials, while each of the generator is used principally just and only for one explosive compound to avoid cross-contamination of the individual thermal vapor phase generator. The calibration system according to the invention is automatically controlled together with actual device just being calibrated by the external computer and the whole calibration procedure is completely robotized, including automatic saving of the calibration results onto the calibrated analytical system flash disk.

### Calibration of the Infra-Red sampler

The properly operating Infra-Red sampler 14 (Fig. 2) provides the important first level of rough pre-separation, using the adequate Infra-Red energy with the homogeneous flow of radiation to release the solid explosive traces from the scanned subject. To achieve the successful operation of the Infra-Red sampler 14 with a well operating collection of explosive traces, the accurate calibration of the sampler/Infra-Red source 5 is necessary.

The special calibration device (Fig. 7) for the Infra-Red source 5 has been developed. According to the invention, it is composed by the Infra-Red flow detection chamber, using the matrix 42 of five units of heat flow sensors 43 (refer to Fig. 7), displaced in the center and surrounding the center of the IR exposed area. The matrix 42 of five heat flow sensors 43 measure the heat flow intensity and area distribution of the flow.

According to the invention, the autonomously operated calibration software of the calibration device automatically sets up the parameters of the Infra-Red sampler 14 to achieve the correct spectrum of the Infra-Red radiation and correct heath flow for proper operation of the sampler 14 and automatically stores the data to the analytical system flash disk 36.

Accordingly, the above description should not be as pertaining only to the precise structures described and illustrated in the accompanying drawings, but rather should be read consistent with and as support to the following claims.

### Legend

- 1: sampler
- 2: thermal decomposition unit
- 3: optical detection unit
- 4: control unit
- 5: infrared radiation source
- 6: radiation element
- 7: black thin-film daze shield
- 8: hood
- 9: aperture
- 10: gas input
- 11: insulation cylinder
- 12: Al/Mgbody
- 13: skin
- 14: IR sampler mirror
- 15: pump
- 16: pump lodgement
- 17: communication unit
- 18: control electronic board
- 19: analytical unit
- 20: detector cartridge
- 21: molecular detection membrane
- 22: annular chamber
- 23: sensing cap
- 24: cartridge body
- 25: optical window
- 24: leading rails
- 27: cup lodgement
- 28: lid
- 29: optical shield
- 30: input/output inlets
- 31: needles line-up
- 32: photomultiplier lamp
- 33: main processor and interface board
- 34: power, charging and communication board
- 35: screen
- 36: flash disk
- 37: power unit
- 38: battery pack
- 39: battery connectors
- 40: control button
- 41: instrument frame
- 42: areal matrix
- 43: sensors
- 44: USB and RS232 ports
- 45: WIFI module
- 46: holster

## Claims

1. A pocket size explosive detector comprising
b.) a sampler (1) having a flat infrared radiation source (5) for evaporating a solid explosive sample from a surface of a scanned subject,
c.) a thermal decomposition unit (2) having a gas input (10) located in the center of the flat infrared radiation source (5) and a silica-glass tube equipped with a heating element for decomposing the evaporated sample into molecular fragments,
d.) a detector cartridge (20) having a main cartridge body (24) with a detection liquid inside and comprising a molecular detection membrane (21) exposed on one side to the molecular fragments and fully sunk under a level of the detection liquid on the other side, the detection liquid generating photons as a response to the molecular fragments coming into reaction with the detection liquid,
e.) a single photon optical detection unit (3) in optical communication with the detector liquid by means of an optical window (25) in the detector cartridge (20) to provide a data stream indicative of explosive molecular fragments' presence in the explosive sample,
f.) a system computer (33 - 35) for further processing the digital output of the single photon optical detection unit (3).

2. The pocket size explosive detector according to claim 1, wherein the sampler (1) comprising the flat infrared radiation source (5) is configured for emitting infrared energy of the infrared wavelength homogeneously from its flat surface to the scanned subject, said infrared radiation source (5) being created by a helix-shaped infrared radiation element (6) with gradient-compensation loops at a lower part, said helix-shaped infrared radiation element (6) being covered by a two-layers black thin-film daze shield (7), emitting mainly infrared radiation, said black thin-film daze shield (7) having a central circular aperture (9) for interposition of the sampling silica glass tube inlet, the completed formation of the said parts being integrated into the one body with a temperature insulating cylinder (11) housed in moulded aluminium/magnesium body (12), said flat infrared radiation source (5) being equipped with cone-shaped hood (8) made of a highly temperature resistant plastic, said cone-shaped hood (8) being equipped with highly polished stainless-steel cylindrical mirror (14) for homogeneous and effective infrared radiation.

3. The pocket size explosive detector according to claim 1, comprising the thermal decomposition unit (2) having the gas input (10) located in the centre of the flat infrared radiation source (5), being composed by a decomposition silica-glass tube, said silica-glass tube being equipped with a non-linear heating element located at a silica-glass tube outer surface in the middle of the silica-glass tube length, said heating element being composed by a heating wire wound with non-linear spacing to compensate for temperature losses at both ends of the silica-glass tube and for securing gradient-less distribution of the operating temperature along the length of the decomposition silica-glass tube, said heating element being driven by pulse-width modulation, PWM, driver to achieve effective power management and accurate temperature measurement, said heating wire is used as a temperature sensing element with the temperature precisely measured by measuring a resistance of the heating wire during the pauses of the PWM power cycle, while the temperature of the heating wire is almost identical with the temperature of the inner surface of the decomposition silica-glass tube due to tight temperature coupling of the heating wire to the decomposition silica-glass tube, said heating element with said defined non-linear heating element is fixed to the outer silica-glass tube surface by a special high temperature resistant cement, the said formation of the thermal decomposition unit (2) is housed inside the said temperature insulating cylinder (11) and having the gas input (10) located in the centre of the flat infrared radiation source (5).

4. The pocket size explosive detector according to claim 1, wherein the cartridge body (24) being made of a transparent and highly pH resistant plastic, having a cylindrical cap lodgement (27) and triangular leading rails (26) for the sensing cap (23) together with the rim, created as an extension of the said cap lodgement (27) for the moulded molecular detection membrane (21), said cartridge body (24) having also a polished optical window (25), all integrated into the cartridge body (24), said cartridge body also having the black rear rectangular plastic lid (28) with a pulling handle located at the rear side of the said cartridge body (24), said detector cartridge (20) having also the sensing cap (23) made of flexible plastics, embattled from the left side on the cylindrical detection cap lodgement (27) of the detector cartridge (20), with proper position given by the triangular leading rails (26), said sensing cap (23) containing two input/output inlets (30) for connecting gas input/output connection needle line-up (31), an annular reaction chamber (22) for the analysed gas circulating around the outer left dry surface of the molecular detection membrane (21) and cylindrical sealing part fitted with negative diameter tolerance to the cylindrical cap lodgement (27) of the detector cartridge (20).

5. The pocket size explosive detector according to claim 1, wherein the single photon optical detection unit (3) having a photomultiplier lamp (32), a sensing analogue electronics, a high voltage source and a control processor, said control processor running a program being created to provide a signal processing converting the photomultiplier lamp (32) signal to a digital data stream with a digital noise reduction procedure and compensation for the photomultiplier lamp's non-linear operation characteristics and a temperature dependences and automatic base-line handling.

6. The pocket size explosive detector according to claim 1, further comprising battery connectors (39) for connecting an exchangeable battery pack (38).

7. The pocket size explosive detector according to claim 1, wherein the explosive detector further comprises a stored calibration data for either quantitative and qualitative analyses of the explosive sample, created by an automatic calibration device.

8. The pocket size explosive detector according to claim 7, wherein the automatic calibration device using the principle of computer controlled thermal vapour phase generators, creating defined flow of defined concentration of the explosive sample in defined number of consequent steps of concentrations, covering thus the whole dynamic range of the detection and analytical explosive detector.

9. The pocket size explosive detector according to claim 8, wherein the multiple thermal vapour phase generators are consequently used to cover the whole wide spectrum calibration for all the existing explosive materials, while each of the generator is used principally just and only for one explosive compound to avoid cross-contamination of the individual thermal vapour phase generator.

10. The pocket size explosive detector with fast response of claim 1, wherein the sampler (1) provides a first level of pre-separation using the appropriate infrared energy with homogeneous radiation to release the solid explosive samples from the scanned subject.

11. The pocket size explosive detector according to claim 1, wherein the explosive detector has a case designed to fit organically to an operator's hand.

12. The pocket size explosive detector according to claim 1, wherein the explosive detector is designed to be operated with just one control button (40).

13. A method for detecting explosives using the pocket size detector of claim 1, comprising
a.) evaporating solid explosive samples from a surface of a scanned subject using an infrared radiation emitted by the flat infrared radiation source (5),
b.) thermally decomposing inside the thermal decomposition unit (2) the gas sample obtained by the evaporation,
c.) using the single photon optical detection unit (3) for detecting photons generated as a response to the molecular fragments coming into reaction with the detection liquid, the single photon optical detection unit (3) providing a data stream indicative of explosive molecular fragments' presence in the sample,
d.) further processing of the digital output of the single photon detection unit by the system computer (33 - 35).

## Patentansprüche

1. Ein Sprengstoffdetektor im Taschenformat umfassend
a.) einen Probenehmer (1) mit einer flachen Infrarot-Strahlungsquelle (5) zum Verdampfen einer festen Explosionsprobe von der Oberfläche eines abgetasteten Subjekts,
b.) eine thermische Zersetzungseinheit (2) mit einem Gaseinlass (10), der sich im Zentrum der flachen Infrarot-Strahlungsquelle (5) befindet, und einem Quarzglasrohr, das mit einem Heizelement zur Zersetzung der verdampften Probe in Molekülfragmente ausgestattet ist,
c.) eine Detektorpatrone (20), die einen Patronenhauptkörper (24) mit einer Detektionsflüssigkeit im Inneren aufweist und eine molekulare Detektionsmembran (21) umfasst, die auf einer Seite den Molekülfragmenten ausgesetzt ist und auf der anderen Seite vollständig unter einen Pegel der Detektionsflüssigkeit gesunken ist, wobei die Detektionsflüssigkeit als Reaktion auf die Molekülfragmente, die mit der Detektionsflüssigkeit reagieren, Photonen erzeugt,
d.) eine optische Einzelphotonen-Detektoreinheit (3) in optischer Kommunikation mit der Detektorflüssigkeit mittels eines optischen Fensters (25) in der Detektorkartusche (20), um einen Datenstrom zu liefern, der das Vorhandensein von explosiven Molekülfragmenten in der Sprengstoffprobe anzeigt,
e.) einen Systemcomputer (33 - 35) zur Weiterverarbeitung der digitalen Ausgabe der optischen Einzelphotonen-Detektoreinheit (3).

2. Sprengstoffdetektor im Taschenformat nach Anspruch 1, wobei der Probenehmer (1), der die flache Infrarot-Strahlungsquelle (5) umfasst, so konfiguriert ist, dass er Infrarotenergie der Infrarot-Wellenlänge homogen von seiner flachen Oberfläche zu dem abgetasteten Subjekt emittiert, wobei die Infrarot-Strahlungsquelle (5) durch ein spiralförmiges Infrarot-Strahlungselement (6) mit Gradienten-Kompensationsschleifen an einem unteren Teil gebildet ist, wobei das spiralförmige Infrarot-Strahlungselement (6) durch einen zweischichtigen schwarzen Dünnfilm-Dämmerungsschirm (7) bedeckt ist, der hauptsächlich Infrarot-Strahlung emittiert, wobei der schwarze Dünnfilm-Dämmerungsschirm (7) eine zentrale kreisförmige Öffnung (9) zur Zwischenanordnung des Probenentnahme-Quarzglasrohreinlasses aufweist, wobei die vollständige Ausbildung der Teile in den einen Körper mit einem temperaturisolierenden Zylinder (11) integriert ist, der in einem geformten Aluminium/Magnesium-Körper (12) untergebracht ist, wobei die flache Infrarot-Strahlungsquelle (5) mit einer kegelförmigen Haube (8) aus einem hochtemperaturbeständigen Kunststoff ausgestattet ist, wobei die kegelförmige Haube (8) mit einem hochpolierten zylindrischen Spiegel (14) aus Edelstahl für homogene und effektive Infrarotstrahlung ausgestattet ist.

3. Sprengstoffdetektor im Taschenformat nach Anspruch 1, umfassend die thermische Zersetzungseinheit (2), deren Gaseinlass (10) sich in der Mitte der flachen Infrarot-Strahlungsquelle (5) befindet und die aus einem Zersetzungsquarzglasrohr besteht, wobei das Quarzglasrohr mit einem nichtlinearen Heizelement ausgestattet ist, das sich an einer Quarzglasrohraussenfläche in der Mitte der Quarzglasrohrlänge befindet, wobei das Heizelement aus einem Heizdraht besteht, der mit nichtlinearem Abstand gewickelt ist, um Temperaturverluste an beiden Enden des Quarzglasrohrs zu kompensieren und um eine gradientenfreie Verteilung der Betriebstemperatur entlang der Länge des Zersetzungsquarzglasrohrs sicherzustellen, wobei das Heizelement durch einen Pulsbreitenmodulationstreiber, PWM-Treiber, angesteuert wird, um ein effektives Leistungsmanagement und eine genaue Temperaturmessung zu erreichen, der genannte Heizdraht als Temperaturfühlerelement verwendet wird, wobei die Temperatur durch Messen eines Widerstands des Heizdrahts während der Pausen des PWM-Leistungszyklus genau gemessen wird, während die Temperatur des Heizdrahts aufgrund der engen Temperaturkopplung des Heizdrahts an das sich zersetzende Quarzglasrohr nahezu identisch mit der Temperatur der Innenfläche des Zersetzungsquarzglasrohrs ist, das Heizelement mit dem definierten nichtlinearen Heizelement durch einen speziellen hochtemperaturbeständigen Zement an der äußeren Oberfläche des Quarzglasrohrs befestigt ist, wobei die Ausbildung der thermischen Zersetzungseinheit (2) innerhalb des temperaturisolierenden Zylinders (11) untergebracht ist und der Gaseinlass (10) in der Mitte der flachen Infrarotstrahlungsquelle (5) angeordnet ist.

4. Sprengstoffdetektor im Taschenformat nach Anspruch 1, bei dem der Patronenkörper (24) aus einem transparenten und hoch pH-beständigen Kunststoff hergestellt ist, der eine zylindrische Kappenaufnahme (27) und dreieckige Führungsschienen (26) für die Sensorkappe (23) zusammen mit dem Rand aufweist, die als eine Verlängerung der Kappenaufnahme (27) für die geformte molekulare Detektionsmembran (21) ausgebildet ist, wobei der Patronenkörper (24) auch ein poliertes optisches Fenster (25) aufweist, die alle in den Patronenkörper (24) integriert sind, wobei der Patronenkörper auch den schwarzen hinteren rechteckigen Kunststoffdeckel (28) mit einem Zuggriff aufweist, der an der Rückseite des Patronenkörpers (24) angeordnet ist, wobei die Detektorkartusche (20) auch die Sensorkappe (23) aus flexiblem Kunststoff aufweist, die von der linken Seite auf der zylindrischen Detektorkappenaufnahme (27) der Detektorkartusche (20) ummantelt ist, wobei die richtige Position durch die dreieckigen Führungsschienen (26) gegeben ist, wobei die Sensorkappe (23) zwei Eingangs-/Ausgangseinlässe (30) zum Verbinden der Nadellinie (31) der Gasein-/Ausgangsverbindung enthält, eine ringförmige Reaktionskammer (22) für das analysierte Gas, die um die äußere linke trockene Oberfläche der molekularen Detektionsmembran (21) und ein zylindrisches Dichtungsteil zirkuliert, das mit negativer Durchmessertoleranz an der zylindrischen Kappenaufnahme (27) der Detektorkartusche (20) angebracht ist.

5. Sprengstoffdetektor im Taschenformat nach Anspruch 1, bei dem die optische Einzelphotonen-Detektoreinheit (3) eine Photovervielfacherlampe (32), eine analoge Erfassungselektronik, eine Hochspannungsquelle und einen Steuerprozessor aufweist, wobei der Steuerprozessor ein Programm ausführt, das erstellt wurde, um eine Signalverarbeitung bereitzustellen, die das Signal der Photovervielfacherlampe (32) in einen digitalen Datenstrom mit einem digitalen Rauschverminderungsverfahren und einer Kompensation der nichtlinearen Betriebscharakteristik der Photovervielfacherlampe und einer Temperaturabhängigkeit und einer automatischen Grundlinienbehandlung umwandelt.

6. Sprengstoffdetektor im Taschenformat nach Anspruch 1, ferner umfassend Batterieanschlüsse (39) zum Anschluss eines austauschbaren Batteriepacks (38).

7. Der Sprengstoffdetektor im Taschenformat nach Anspruch 1, wobei der Sprengstoffdetektor ferner gespeicherte Kalibrierdaten für entweder quantitative oder qualitative Analysen der Sprengstoffprobe umfasst, die durch eine automatische Kalibriervorrichtung ausgebildet ist.

8. Sprengstoffdetektor im Taschenformat nach Anspruch 7, bei dem die automatische Kalibriervorrichtung das Prinzip computergesteuerter thermischer Dampfphasengeneratoren anwendet, die einen definierten Fluss einer definierten Konzentration der Sprengstoffprobe in einer definierten Anzahl aufeinanderfolgender Konzentrationsschritte erzeugen und somit den gesamten dynamischen Bereich des Detektors und des analytischen Sprengstoffdetektors abdecken.

9. Sprengstoffdetektor im Taschenformat nach Anspruch 8, bei dem die mehrfachen thermischen Dampfphasengeneratoren in der Folge dazu verwendet werden, das gesamte breite Kalibrierungsspektrum für alle vorhandenen Sprengstoffe abzudecken, während jeder der Generatoren im Prinzip nur und nur für eine Sprengstoffverbindung verwendet wird, um eine Kreuzkontamination des einzelnen thermischen Dampfphasengenerators zu vermeiden.

10. Der Sprengstoffdetektor im Taschenformat mit schnellem Ansprechverhalten nach Anspruch 1, wobei der Probennehmer (1) eine erste Stufe der Vortrennung unter Verwendung der geeigneten Infrarotenergie mit der homogenen Strahlung liefert, um die festen Sprengstoffproben vom abgetasteten Objekt freizusetzen.

11. Sprengstoffdetektor im Taschenformat nach Anspruch 1, wobei der Sprengstoffdetektor ein Gehäuse hat, das so konstruiert ist, dass es organisch in die Hand eines Bedieners passt.

12. Der Sprengstoffdetektor im Taschenformat nach Anspruch 1, wobei der Sprengstoffdetektor so ausgelegt ist, dass er mit nur einem Steuerknopf (40) bedient werden kann.

13. Verfahren zum Detektieren von Sprengstoffen unter Verwendung des Detektors im Taschenformat nach Anspruch 1, umfassend
a.) Verdampfen von festen Sprengstoffproben von einer Oberfläche eines abgetasteten Objekts unter Verwendung einer von der flachen Infrarotstrahlungsquelle (5) emittierten Infrarotstrahlung,
b.) thermische Zersetzung der durch die Verdampfung erhaltenen Gasprobe innerhalb der thermischen Zersetzungseinheit (2),
c.) Verwendung der optischen Einzelphotonen-Detektoreinheit (3) zum Nachweis von Photonen, die als Reaktion auf die Molekülfragmente erzeugt werden, die mit der Nachweisflüssigkeit in Reaktion treten, wobei die optische Einzelphotonen-Detektoreinheit (3) einen Datenstrom liefert, der das Vorhandensein explosiver Molekülfragmente in der Probe anzeigt,
d.) Weiterverarbeitung der digitalen Ausgabe der Einzelphotonen-Detektionseinheit durch den Systemcomputer (33 - 35).

## Revendications

1. Détecteur d'explosif de poche comprenant
b.) un échantillonneur (1) ayant une source de rayonnement infrarouge plate (5) pour évaporer un échantillon d'explosif solide à partir d'une surface d'un sujet balayé,
c.) une unité de décomposition thermique (2) ayant une entrée de gaz (10) située au centre de la source de rayonnement infrarouge plate (5) et un tube en verre de silice équipé d'un élément chauffant pour décomposer l'échantillon évaporé en fragments moléculaires,
d.) une cartouche de détection (20) ayant un corps de cartouche principal (24) avec un liquide de détection à l'intérieur et comprenant une membrane de détection moléculaire (21) exposée d'un côté aux fragments moléculaires et entièrement enfoncée sous un niveau du liquide de détection de l'autre côté, le liquide de détection générant des photons en réponse aux fragments moléculaires entrant en réaction avec le liquide de détection,
e.) une unité de détection optique à photon unique (3) en communication optique avec le liquide de détection au moyen d'une fenêtre optique (25) dans la cartouche de détection (20) pour fournir un flux de données indiquant la présence de fragments moléculaires explosifs dans l'échantillon d'explosif,
f.) un ordinateur de système (33 - 35) pour un traitement ultérieur de la sortie numérique de l'unité de détection optique à photon unique (3).

2. Détecteur d'explosif de poche selon la revendication 1, dans lequel l'échantillonneur (1) comprenant la source de rayonnement infrarouge plate (5) est configuré pour émettre de l'énergie infrarouge de la longueur d'onde infrarouge de manière homogène depuis sa surface plate vers le sujet balayé, ladite source rayonnement infrarouge (5) étant créée par un élément de rayonnement infrarouge en forme d'hélice (6) avec des boucles de compensation de gradient à une partie inférieure, ledit élément de rayonnement infrarouge en forme d'hélice (6) étant recouvert par un écran anti-étourdissement à couche mince noire à deux couches (7), émettant principalement un rayonnement infrarouge, ledit écran anti-étourdissement à couche mince noire (7) ayant une ouverture circulaire centrale (9) pour l'interposition de l'entrée du tube de verre de silice d'échantillonnage, la formation terminée desdites parties étant intégrée dans le corps unique avec un cylindre d'isolation thermique (11) logé dans un corps moulé en aluminium / magnésium (12), ladite source de rayonnement infrarouge plat (5) étant équipée d'un capot en forme de cône (8) en plastique hautement résistant à la température, ledit capot en forme de cône (8) étant équipé d'un miroir cylindrique en acier inoxydable hautement poli (14) pour un rayonnement infrarouge homogène et efficace.

3. Détecteur d'explosif de poche selon la revendication 1, comprenant l'unité de décomposition thermique (2) ayant l'entrée de gaz (10) située au centre de la source de rayonnement infrarouge plate (5), étant composée d'un tube de décomposition en verre de silice, ledit tube en verre de silice étant équipé d'un élément chauffant non linéaire situé sur une surface extérieure du tube en verre de silice au milieu de la longueur du tube en verre de silice, ledit élément chauffant étant composé d'un fil chauffant enroulé avec un espacement non linéaire pour compenser les pertes de température aux deux extrémités du tube de verre de silice et pour assurer une distribution sans gradient de la température de fonctionnement sur la longueur du tube de décomposition en verre de silice, ledit élément chauffant étant entraîné par un pilote de modulation de largeur d'impulsion, PWM, pour obtenir une gestion efficace de l'énergie et une mesure précise de la température, ledit fil chauffant est utilisé comme élément de détection de température, la température étant mesurée avec précision en mesurant une résistance du fil chauffant durant les pauses du cycle d'alimentation PWM, tandis que la température du fil chauffant est presque identique à la température de la surface intérieure du tube de décomposition en verre de silice en raison du couplage de température étroit du fil chauffant au tube de décomposition en verre de silice, ledit élément chauffant avec ledit élément chauffant non linéaire défini est fixé à la surface extérieure du tube de verre de silice par un ciment spécial résistant aux hautes températures, ladite formation de l'unité de décomposition thermique (2) est logée à l'intérieur dudit cylindre d'isolation thermique (11) et ayant l'entrée de gaz (10) située au centre de la source de rayonnement infrarouge plate (5).

4. Détecteur d'explosif de poche selon la revendication 1, dans lequel le corps de cartouche (24) est fait d'un plastique transparent et hautement résistant au pH, ayant un logement de capuchon cylindrique (27) et des rails avant triangulaires (26) pour le capuchon de détection (23) avec le rebord, créé comme une extension dudit logement de capuchon (27) pour la membrane de détection moléculaire moulée (21), ledit corps de cartouche (24) ayant également une fenêtre optique polie (25), le tout intégré dans le corps de cartouche (24), ledit corps de cartouche ayant également le couvercle en plastique rectangulaire arrière noir (28) avec une poignée de traction située sur le côté arrière dudit corps de cartouche (24), ladite cartouche de détection (20) ayant également le capuchon de détection (23) en matière plastique flexible, emboîtée du côté gauche sur le logement de capuchon de détection cylindrique (27) de la cartouche de détection (20), avec une position correcte donnée par les rails de guidage triangulaires (26), ledit capuchon de détection (23) contenant deux entrées d'entrées et de sortie (30) pour connecter l'entrée de gaz / une ligne d'aiguilles de connexion de sortie (31), une chambre de réaction annulaire (22) pour le gaz analysé circulant autour de la surface sèche externe gauche de la membrane de détection moléculaire (21) et une partie d'étanchéité cylindrique équipée d'une tolérance de diamètre négative par rapport au capuchon cylindrique logement (27) de la cartouche de détection (20).

5. Détecteur d'explosif de poche selon la revendication 1, dans lequel l'unité de détection optique à photon unique (3) ayant une lampe photomultiplicatrice (32), une électronique de détection analogique, une source haute tension et un processeur de commande, ledit processeur de commande exécutant un programme étant créé pour fournir un traitement de signal convertissant le signal de la lampe photomultiplicatrice (32) en un flux de données numériques avec une procédure de réduction de bruit numérique et une compensation des caractéristiques de fonctionnement non linéaires de la lampe photomultiplicateur et des dépendances de température et une gestion automatique de la ligne de base.

6. Détecteur d'explosif de poche selon la revendication 1, comprenant en outre des connecteurs de batterie (39) pour connecter un bloc de batterie échangeable (38).

7. Détecteur d'explosif de poche selon la revendication 1, dans lequel le détecteur d'explosif comprend en outre des données d'étalonnage stockées pour des analyses quantitatives et qualitatives de l'échantillon d'explosif, créées par un dispositif d'étalonnage automatique.

8. Détecteur d'explosif de poche selon la revendication 7, dans lequel le dispositif d'étalonnage automatique utilisant le principe des générateurs de phase vapeur thermique commandés par ordinateur, créant un flux défini de concentration définie de l'échantillon explosif en un nombre défini d'étapes consécutives de concentrations, couvrant ainsi toute la plage dynamique du détecteur d'explosif de détection et d'analyse.

9. Détecteur d'explosif de poche selon la revendication 8, dans lequel les multiples générateurs thermiques de phase vapeur sont par conséquent utilisés pour couvrir tout l'étalonnage à large spectre pour tous les matériaux explosifs existants, tandis que chacun des générateurs est utilisé principalement juste et uniquement pour un composé explosif pour éviter la contamination croisée du générateur thermique de phase vapeur individuel.

10. Détecteur d'explosif de poche à réponse rapide selon la revendication 1, dans lequel l'échantillonneur (1) fournit un premier niveau de pré-séparation en utilisant l'énergie infrarouge appropriée avec un rayonnement homogène pour libérer les échantillons d'explosifs solides du sujet balayé.

11. Détecteur d'explosif de poche selon la revendication 1, dans lequel le détecteur d'explosif a un boîtier conçu pour s'adapter de manière organique à la main d'un opérateur.

12. Détecteur d'explosif de poche selon la revendication 1, dans lequel le détecteur d'explosif est conçu pour fonctionner avec un seul bouton de commande (40).

13. Procédé de détection d'explosifs utilisant le détecteur de poche selon la revendication 1, comprenant
a.) l'évaporation d'échantillons d'explosifs solides d'une surface d'un sujet balayé en utilisant un rayonnement infrarouge émis par la source de rayonnement infrarouge plate (5),
b.) la décomposition thermique à l'intérieur de l'unité de décomposition thermique (2) de l'échantillon de gaz obtenu par évaporation,
c.) l'utilisation de l'unité de détection optique à photon unique (3) pour détecter les photons générés en réponse aux fragments moléculaires entrant en réaction avec le liquide de détection, l'unité de détection optique à photon unique (3) fournissant un flux de données indicatif de présence de fragments moléculaires explosifs dans l'échantillon,
d.) le traitement supplémentaire de la sortie numérique de l'unité de détection de photon unique par l'ordinateur de système (33 - 35).
